# EUROPEAN PATENT APPLICATION

(11) **EP 2 468 718 A1**
(43) Date of publication of application: **27.06.2012**
(21) Application number: 10306485.3
(22) Date of filing: 22.12.2010
(51) Int. Cl.: C07C 277/08, C07C 279/12, C07C 279/22, A61K 31/155, A61P 31/10

(54) **Cabanillasin, a new antifungal compound, produced by entomopathogenic xenorhabdus cabanillasii**

(71) Applicant: Nosopharm, 30035 Nimes (FR); INSTITUT NATIONAL DE LA RECHERCHE AGRONOMIQUE, 75007 Paris (FR)
(72) Inventor: Gualtieri, Maxime, 30660 Gallargues-le-Montueux (FR); Villain-Guillot, Philippe, 30000 Nimes (FR); Givaudan, Alain, 34270 Saint-Mathieu-de-Treviers (FR); Pages, Sylvie, 34980 Saint-Clement-de-Riviere (FR)
(74) Representative: Tetaz, Franck Claude Edouard

(57) **Abstract**

*Xenorhabdus cabanillasii* strain deposited at CNCM having the accession number CNCM I-4418. Antifungal compound purified from *Xenorhabdus cabanillasii* strain CNCM I-4418 and methods for producing said compound and pharmaceutical composition comprising said compound and a pharmaceutically acceptable carrier for use in the treatment of fungal disease.

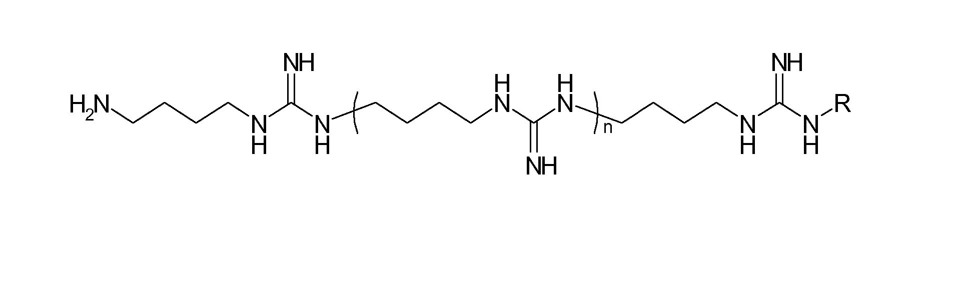

## Description

The present invention relates to a new antifungal compound, to a strain of *Xenorhabdus cabanillasii* producing this compound and to the use of this compound in the treatment of fungal disease in humans and animals.

Since the early 1980s, fungi have emerged as major causes of human disease, especially among the immunocompromised and those hospitalized with serious underlying diseases (Pfaller M.A. et al, Clinical Microbiology Reviews, January 2007, p. 133-163, Vol. 20, No. 1). A recent study of the epidemiology of sepsis found that the annual number of cases of sepsis caused by fungal organisms in the United States increased by 207% between 1979 and 2000. The morbidity and mortality associated with these infections are substantial, and it is clear that fungal diseases have emerged as important public health problems. The majority of nosocomial fungal infections are caused by *Candida* species, with *Candida albicans* being the most common etiological agent of fungal bloodstream infections. *Candida* and *Aspergillus* infections represent 90 % of all nosocomial fungal infection. *Cryptococcus neoformans* has become also a major opportunistic pathogen in immunocompromised individuals (Perea S. et al, Clinical infectious diseases 2002; 35, 1073-80). The number of classes of antifungal used in therapy is relatively low (echinocandin, amphotericin B, azole derivatives and flucytosin). Moreover, the incidence of antifungal resistance is on the increase worldwide. The search for new antifungal compounds is necessary to ensure that fungal infections can be effectively treated in the future. Bacteria remain a major resource to discover new molecules.

In our research program, we worked an understudied bacterial genus: *Xenorhabdus.* Gram-negative bacterial strains of the genus *Xenorhabdus* are known to be symbiotically associated with soil dwelling nematodes of the *Steinernematidae* family. After entering the insect larvae via natural openings, nematodes release bacteria from their intestine to the host's hemocoel. Bacteria are involved in killing the insect host by producing insecticidal proteins and inhibitors of the insect immune system. The bacteria proliferate in the killed host and favour the reproduction of the nematode by degrading the insect biomass and by producing antibiotics that inhibits the development of the other microorganisms present in the insect corpse (bacteria, fungi). Only a few families of antifungal compounds have been described from *Xenorhabdus* in the literature: xenorhabdins (Rhodes S.H. et al. , WO 84/01775; McInerney B.V. et al, J. Nat. Prod. 54, 785-95 (1991)), indole derivatives (Webster J.M. et al, US patent 5,569,668; Li J. Et al, J. Nat. Prod. 58, 1081-6 (1995); Li J. et al, Can. J. Microbiol. 43, 770-3 (1997)), and PAX (Gualtieri M. et al, J.Antibiot. 62(6) 295-302 (2009)).

Cabinallisin is a new antifungal compound produced by *Xenorhabdus cabanillasii* strain JM26 (CNCM I-4418) which shows activity against yeasts and filamentous fungi involved in hospital-acquired infections.

### Summary

A first object of the present invention is the *Xenorhabdus cabanillasii* strain deposited at CNCM on 15 December 2010 having the accession number CNCM I-4418.

Another object of the present invention is the culture supernatant from the *Xenorhabdus cabanillasii* strain according to the invention having antifungal activity.

Another object of the present invention is an extract from the *Xenorhabdus cabanillasii* strain of the present invention having antifungal activity.

The present invention is also related to a compound of formula (I): 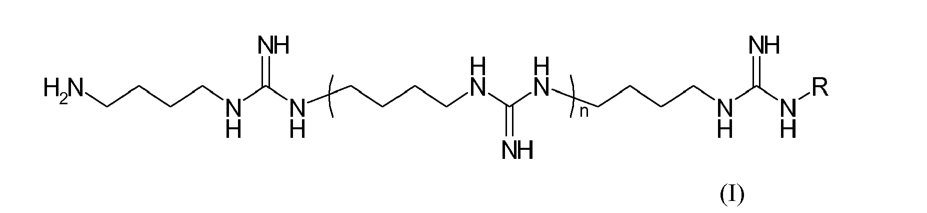 wherein
n is 1, 2, 3 or 4,
R is selected in the group consisting of H, C(=O)-CH₃, C(=O)-CH₂-CH₃ and C(=O)-CH(-CH3)-CH3.

In preferred embodiments R is C(=O)-CH₃. Preferably n = 2.

The present invention is also related to a compound of formula (II):

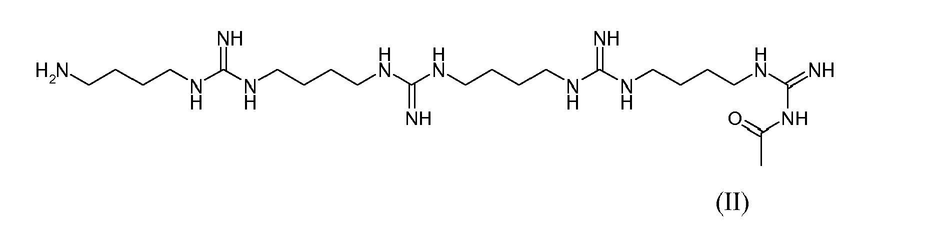

Preferably, the compounds of the present invention are for use as a medicament.

More preferably, the compounds of the present invention are for use as an antifungal agent.

Even more preferably, the compounds of the present invention are for use in treatment of fungal disease caused by yeast and/or filamentous fungi.

The present invention is also directed to the compounds of formula (I) for use in treatment of yeast and/or fungal disease caused by a pathogen selected from *Candida*, *Pneumocystis*, *Cryptococcus*, *Trichosporon*, *Aspergillus*, *Exophiala, Fonsecaea, Phialophora*, *Geotrichum*, *Pseudallescheria*, *Fusarium*, *Rhizopus*, *Epidermophyton*, *Microsporum*, *Trichophyton*, *Coccidioides* and *Histoplasma*.

In a preferred embodiment, the compounds of formula (I) are for use in treatment of yeast and/or fungal disease caused by a pathogen selected from *C.albicans*, *C.glabrata*, *C.krusei*, *C.lusitaniae*, *Cr.neoformans*, *A.fumigatus*, *R.oryzae* and *F.oxysporum.*

Another embodiment of the present invention is a pharmaceutical composition comprising:
- an effective amount of a compound of formula (I) 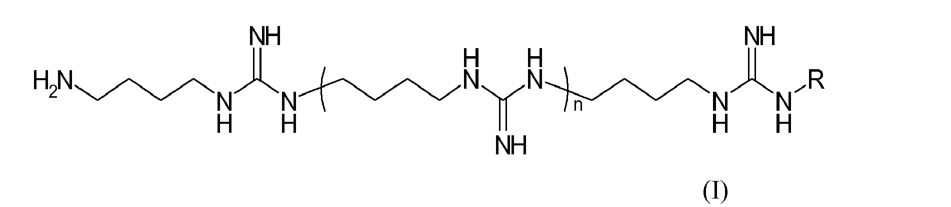 wherein
   n is 1, 2, 3 or 4,
   R is selected in the group consisting of H, C(=O)-CH₃, C(=O)-CH₂-CH₃ and C(=O)-CH(-CH₃)-CH₃.
   - a pharmaceutically acceptable carrier.

In preferred embodiments R is C(=O)-CH₃. Preferably n = 2. Even more preferably, the compound is the compound of formula (II): 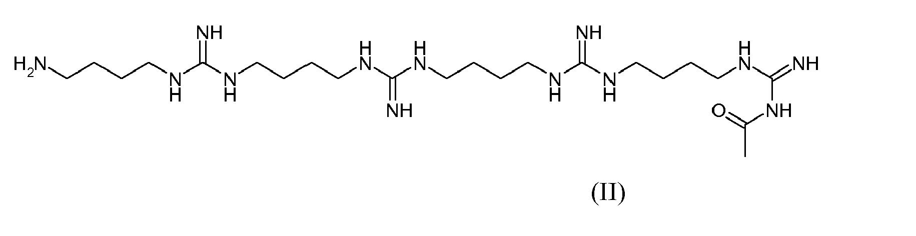 The present invention is also related to a method for producing a compound according to formula (II) comprising the following steps
- Growing *Xenorhabdus cabanillasii* strain deposited at CNCM on 15-12-2010 having the accession number CNCM I-4418 in a liquid culture medium,
- Purifying a compound according to formula (II)

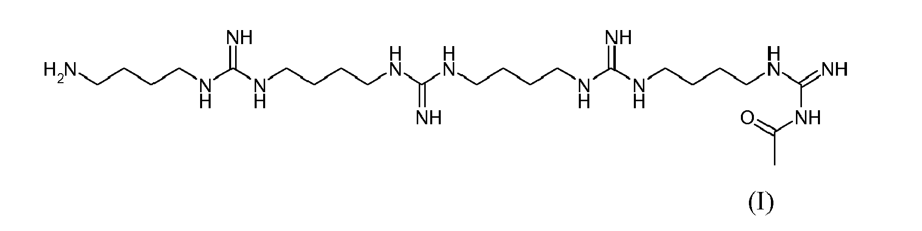

In preferred embodiments, the compound of formula (II) is purified from the culture supernatant after removal of the *Xenorhabdus cabanillasii* cells.

### Detailed description of the invention

In a first aspect, the present invention is related to *Xenorhabdus cabanillasii* strain deposited in the name of Institut National de la Recherche Agronomique (INRA) at Collection Nationale de Cultures de Microorganismes (CNCM) on 15 December 2010 having the accession number CNCM I-4418.

It has been found that *Xenorhabdus cabanillasii* strain CNCM I-4418 produces a compound showing antifungal activity.

When *Xenorhabdus cabanillasii* strain CNCM I-4418 is grown in a liquid culture medium, the antifungal compound of the present invention is secreted into the culture supernatant. In another aspect, the present invention is related to a culture supernatant from *Xenorhabdus cabanillasii* strain CNCM I-4418 showing antifungal activity. For the preparation of a culture supernatant having antifungal activity *Xenorhabdus cabanillasii* strain CNCM I-4418 is grown in a liquid culture medium under standard conditions, the bacterial cells are removed and the supernatant is recovered. The bacterial cells may for example be removed by centrifugation or filtration.

In another aspect, the invention is related to extracts from *Xenorhabdus cabanillasii* strain CNCM I-4418 showing antifungal activity. Cell extracts from *Xenorhabdus cabanillasii* may be prepared according to any appropriate method known to the skilled person.

The terms "antifungal activity" as used herein refer generally to an effect in which a reduction, inhibition or a halt in the growth of a fungi is achieved. Antifungal activity may be tested according to any known method such as a microdilution method.

*Xenorhabdus cabanillasii* strain CNCM I-4418, culture supernatant from this strain and cell extracts derived from this strain exhibit antifungal activity against different fungi including human fungal pathogens.

Antifungal activity has been demonstrated against ascomycetes yeasts such as yeast of the genus *Candida* including *Candida albicans*, *Candida glabrata*, *Candida krusei* and *Candida lusitaniae.*

Antifungal activity has also been demonstrated against basidiomycetes yeasts such as yeast of the genus *Cryptococcus* including *Cryptococcus neoformans.*

Antifungal activity has also been demonstrated against ascomycetes filamentous fungi such as filamentous fungi of the genus *Aspergillus* including *Aspergilus fumigatus* and filamentous fungi of the genus *Fusarium* including *Fusarium oxysporum.*

Antifungal activity has also been demonstrated against zygomycetes filamentous fungi such as filamentous fungi of the genus *Rhizopus* including *Rhizopus oryzae.*

Compounds having antifungal activity as described above has been purified from the culture supernatant *of Xenorhabdus cabanillasii* strain CNCM I-4418.

In a first embodiment, the present invention is directed to compounds of formula (I): 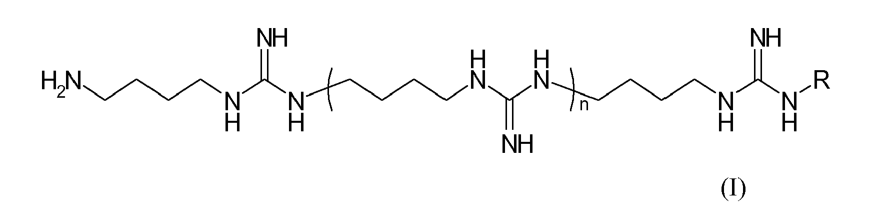 wherein
n is 1, 2, 3 or 4,
R is selected in the group consisting of H, C(=O)-CH₃, C(=O)-CH₂-CH₃ and C(=O)-CH(-CH₃)-CH₃,
a salt, a hydrate or a solvate thereof.

Preferably, R is C(=O)-CH₃. Preferably n=2.

In preferred embodiments, the present invention is related to a compound of formula (I): 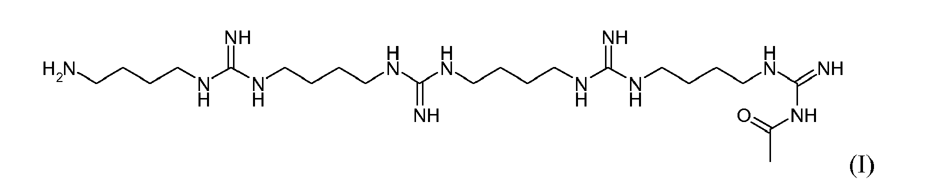 a salt, a hydrate or a solvate thereof. This compound has been named "Cabanillasin".

The term "Cabanillasin" refers to the compound of formula I, a salt, a hydrate or a solvate thereof.

In preferred embodiments, the antifungal compounds according to the present invention are for use as a medicament, for use as an antifungal agent or for use in the treatment of fungal disease in particular of fungal disease caused by yeast and/or filamentous fungi.

The compounds of the present invention are preferably for use in the treatment of fungal infection and in particular in hospital-acquired infections or nosocomial fungal infections.

The invention is also related to the use of compounds of formula (I) for the manufacture of a medicament for treatment of fungal infection or fungal disease.

The present invention also provides methods for treating fungal infection and/or fungal disease including administering an effective amount of compounds of formula (I) to a human or to a patient in need thereof. In a preferred embodiment, the invention relates to methods for treatment of fungal disease and or fungal infection.

In one embodiment, the patient is a mammal, i.e., a living mammal. In one embodiment, the patient is a human, i.e., a living human, including a living human child and a living human adult.

The term "treatment," as used herein in the context of treating a condition, refers generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, amelioration of the condition, and cure of the condition. Treatment as a prophylactic measure (i.e., prophylaxis) is also included. For example, use with subjects who have not yet developed the condition, but who are at risk of developing the condition, is encompassed by the term "treatment."

The term "therapeutically-effective amount," as used herein, refers to the amounts of the active agents (compounds of formula (I) or a salt, hydrate, or solvate thereof) that is effective for producing some desired therapeutic effect, commensurate with a reasonable benefit/risk ratio, when administered in accordance with a desired treatment regimen.

The term "treatment" includes combination treatments and therapies, in which two or more treatments or therapies are combined, for example, sequentially or simultaneously. For example, the active agents of the present invention may also be used in further combination therapies, e.g., in conjunction with other agents, for example, other antifungal agents, etc.

Another aspect of the present invention is a pharmaceutical composition comprising:
- an effective amount of a compound of formula (I) 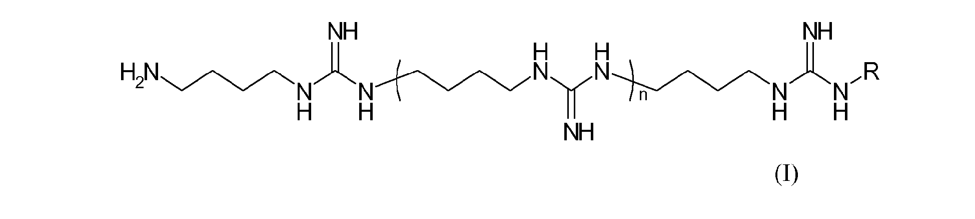 wherein
   n is 1, 2, 3 or 4,
   R is selected in the group consisting of H, C(=O)-CH₃, C(=O)-CH₂-CH₃ and C(=O)-CH(-CH₃)-CH₃, and
- a pharmaceutically acceptable carrier.

Preferably R is C(=O)-CH₃. Preferably n=2. More preferably the compound is a compound according to formula (II):

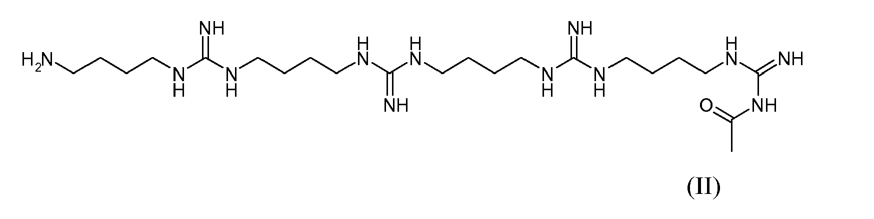

As used herein, "pharmaceutically acceptable carriers" includes any and all solvents, dispersion media, coatings, and the like that are physiologically compatible.

The compositions of the invention may be in a variety of forms. These include for example liquid, semi-solid, and solid dosage forms, but the preferred form depends on the intended mode of administration and therapeutic application.

These pharmaceutical compositions are preferably for oral, topical, percutaneous or parenteral administration.

The compositions as described herein may be orally administered.

As solid compositions for oral administration, tablets, pills, powders (gelatine capsules, sachets) or granules may be used. In these compositions, the active ingredient according to the invention is mixed with one or more inert diluents, such as starch, cellulose, sucrose, lactose or silica, under an argon stream. These compositions may also comprise substances other than diluents, for example one or more lubricants such as magnesium stearate or talc, a coloring, a coating (sugar-coated tablet) or a glaze. Preferred oral compositions include coated and uncoated tablets, hard and soft gelatin capsules, sugar-coated pills, lozenges, pellets, and powders.

As liquid compositions for oral administration, there may be used pharmaceutically acceptable solutions, suspensions, emulsions, syrups and elixirs containing inert diluents such as water, ethanol, glycerol, vegetable oils or paraffin oil. These compositions may comprise substances other than diluents, for example wetting, sweetening, thickening, flavouring or stabilizing products.

In preferred embodiments, the pharmaceutical compositions for oral use contain a compound according to the present invention together with the usual excipients as diluting agents like mannitol, lactose and sorbitol; binding agents like starchs, gelatins, sugars, cellulose derivatives, natural gums and polyvinylpyrrolidone; lubricating agents like talc, stearates, hydrogenated vegetable oils, polyethylenglycol and colloidal silicon dioxide; disintegrating agents like starchs, celluloses, alginates, gums and reticulated polymers; coloring, flavoring and sweetening agents.

In other embodiments, the compositions comprise a compound according to the present invention with carriers or excipients suitable for topical administration. Preferred compositions for topical administration of compounds according to the invention include ointments, pomades, creams, gels, and lotions.

The pharmaceutical compositions for topical use contain a compound of the present invention together with the usual excipients like white petrolatum, white wax, lanolin and derivatives thereof, stearylic alcohol, propylenglycol, sodium lauryl sulfate, ethers of the fatty polyoxyethylene alcohols, sorbitan monostearate, glyceryl monostearate, propylene glycol monostearate, polyetilene glycols, methylcellulose, hydroxymethylpropylcellulose, sodium carboxymethylcellulose, colloidal aluminium and magnesium silicate, sodium alginate. Any topical preparation may be used in the present invention, for instance ointments, pomades, creams, gels and lotions.

The doses of compound according to the present invention depend on the desired effect, the duration of the treatment and the route of administration used.

In preferred embodiments, the pharmaceutical compositions according to the present invention are for use as an antifungal agent or for use in the treatment of fungal disease in particular of fungal disease caused by yeast and/or filamentous fungi.

The pharmaceutical compositions of the present invention are preferably for use in the treatment of fungal infection and in particular in hospital-acquired infections or nosocomial fungal infections.

The compounds and compositions of the present invention are preferably for use in the treatment of disease and/or infection caused by yeast of the genus *Candida* (*Candida albicans*, *Candida glabrata*, *Candida tropicalis*, *Candida krusei* and *Candida lusitaniae*), yeast of the genus *Pneumocystis* (*Pneumocystis jirovecii*), yeast of the genus *Cryptococcus (Cryptococcus neoformans, Cryptococcus laurentii, Cryptococcus albidus, Cryptococcus gattii*), yeast of the genus *Trichosporon*, filamentous fungi of the genus *Aspergillus* (*Aspergillus fumigatus*, *Aspergillus flavus*, *Aspergillus clavatus*), filamentous fungi of the genus *Exophiala* (*Exophiala jeanselmei*), filamentous fungi of the genus *Fonsecaea* (*Fonsecaea pedrosoi*, *Fonsecaea compacta*), filamentous fungi of the genus *Phialophora* (*Phialophora verrucosa*), filamentous fungi of the genus *Geotrichum* (*Geotrichum candidum*), filamentous fungi of the genus *Pseudallescheria* (*Pseudallescheria boydii*), filamentous fungi of the genus *Fusarium* including (*Fusarium oxysporum*), filamentous fungi of the genus *Rhizopus* (*Rhizopus oryzae*), fungi of the genus *Epidermophyton* (*Epidermophyton floccosum*), fungi of the genus *Microsporum* (*Microsporum canis*, *Microsporum audouinii*), fungi of the genus *Trichophyton* (*Trichophyton interdigitale*, *Trichophyton mentagrophytes*, *Trichophyton tonsurans*, *Trichophyton schoenleini*, *Trichophyton rubrum*), fungi of the genus *Coccidioides* (*Coccidioides immitis*, *Coccidioides posadasii*), and fungi of the genus *Histoplasma* (*Histoplasma capsulatum*, *Histoplasma duboisii*).

The compounds of the present invention may be combined with other active compounds exhibiting an antifungal activity. The pharmaceutical compositions encompassed by the present invention may also contain a further therapeutic agent for the treatment of fungal disease or fungal infection.

Another aspect of the present invention is a method for producing a compound according to formula (II) comprising the following steps:
- Growing *Xenorhabdus cabanillasii* strain CNCM I-4418 in a liquid culture medium,
- Purifying a compound according to formula (II):

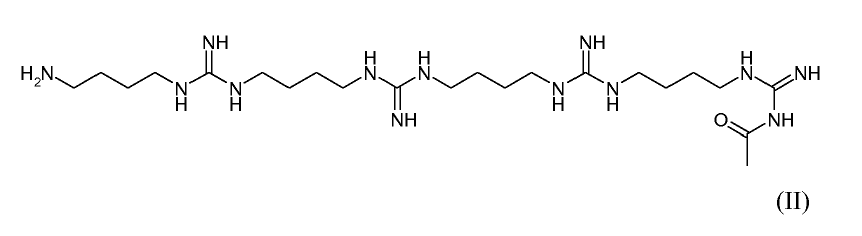

The active compounds according to the present invention may be purified from the *Xenorhabdus cabanillasii* cells of the present invention. Advantageously, the active compounds of the present invention may be purified from the culture supernatant after removal of the *Xenorhabdus cabanillasii* cells. For the preparation of a culture supernatant having antifungal activity *Xenorhabdus cabanillasii* strain CNCM I-4418 is grown in a liquid culture medium under standard conditions, the bacterial cells are removed and the supernatant is recovered. The bacterial cells may for example be removed by centrifugation or filtration.

Further purification of the active compounds according to the present invention may be carried out by any known method including cation-exchange chromatography, reversed-phase chromatography and/or reverse phase HPLC.

In preferred embodiments, the active compounds are purified from the culture supernatant of *Xenorhabdus cabanillasii* strain CNCM I-4418 by successive cation-exchange chromatography, reversed-phase chromatography and reverse phase HPLC.

### Figures

Figure 1: HPLC chromatogram of Cabanillasin
Figure 2: Viability of hTERT HME-1 at 8 concentrations of Cabanillasin
Figure 3: Viability of PC-3 at 8 concentrations of Cabanillasin
Figure 4: ESI-MS spectrum of Cabanillasin

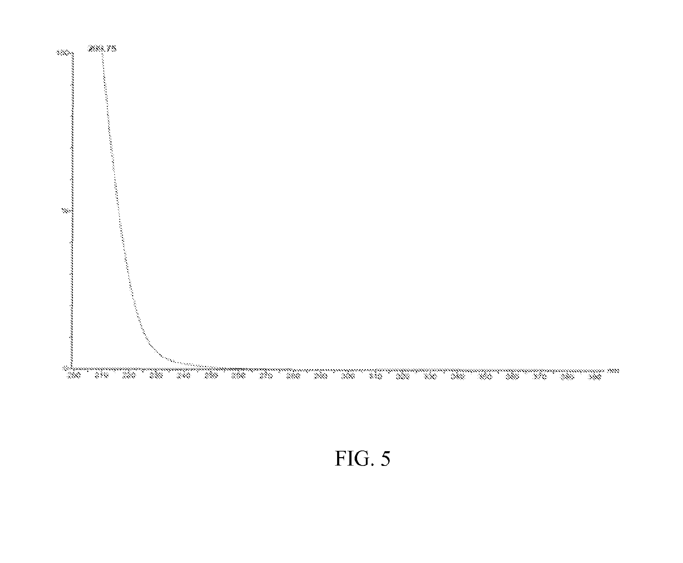

Figure 5: UV-absorption spectrum of Cabanillasin

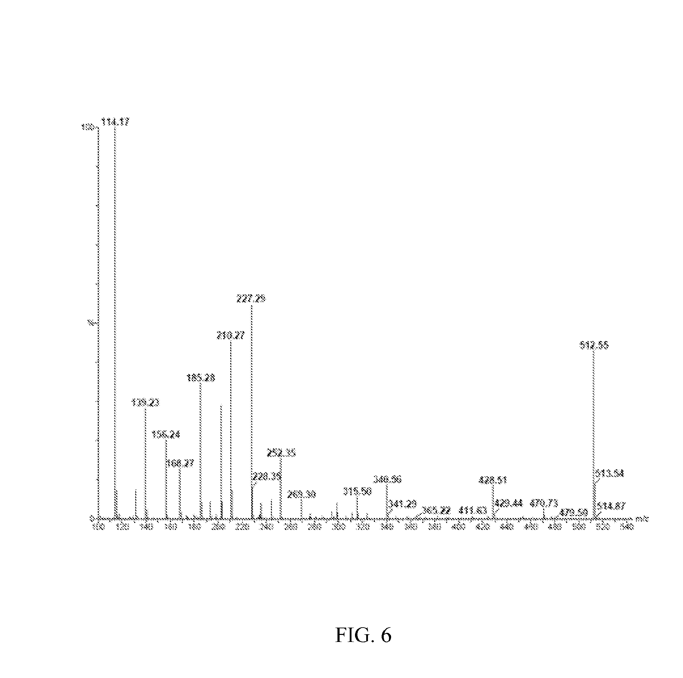

Figure 6: MSMS fragmentation of Cabanillasin

### EXAMPLES

### A. Materials and methods

### Producing organism

*Xenorhabdus cabanillasi* CNCM I-4418 was grown on Luria-Bertani medium (LB, composed of bactotryptone 10 g/L, yeast extract 5 g/L and NaCl 10 g/L) for liquid culture and on LB-agar for solid cultures. The phase status (I or II) of this strain was determined by culturing on NBTA (Nutrient agar (Difco) 31 g/L, bromothymol blue 25 mg/L and 2, 3, 5-triphenyl tetrazolium chloride 1% 40 mg/L) and measuring antibacterial activity against *Micrococcus luteus. Xenorhabdus* exhibit two colony forms or variants when cultured in vitro. Modifications of the outer membrane induce differential adsorption of dyes by variants. Phase I variants absorb dyes and are blue on NBTA plates, while phase II colonies are red. Phases I and II of strains are indicated as suffixes (/1 and /2, respectively) attached to strain designations. This strain was maintained at 15°C on NBTA medium.

### Antifungal susceptibility testing methods

Cabanillasin activity was tested against different fungal human pathogens: ascomycetes yeasts (*Candida albicans* ATCC 90029, *Candida Glabrata* ATCC 90030, *Candida krusei* ATCC 6258 and *Candida lusitaniae* CBS 6936), basidiomycetes yeasts (*Cryptococcus neoformans* clinical strain), ascomycetes filamentous fungi (*Aspergilus fumigatus* clinical strain, *Fusarium oxysporum* clinical strain) and zygomycetes filamentous fungi (*Rhizopus oryzae* environemental strain) by the M38-A microdilution method with RPMI 1640 medium (RPMI 1640, MOPS 160 mM, glucose 18 g/L, pH 7) as recommended in the CLSI M23-A document. The NCCLS M27-A2 broth microdilution method (NCCLS) was used when yeasts were tested.

Yeasts and filamentous inoculi were prepared on YPD medium (yeast extract 10 g/L, peptone 10 g/L and glucose 20 g/L) and Malt medium (malt extract 20 g/L) respectively. The densities of inoculi were adjusted by dilution in RPMI 1640 broth to a value ranged from 5.10² to 5.10³ CFU/mL for yeasts isolates and 5.10³ to 5.10⁴ CFU/mL for filamentous fungi. Microdilution trays (96 U-bottom shaped) containing 190 µL fungal inoculum were added with 10 µL Cabanillasin solution (final concentration ranging from 50 to 0.098 mg/L for all fungi). After inoculation of the trays, all microdilution trays were incubated at 35°C in ambient air. The culture absorbance was measured at 600 nm, 24 h and 48 h after the beginning of the experiments for the yeasts and 48 h and 72 h after the beginning of the experiments for filamentous fungi and *Cryptococcus neoformans.* The activity results correspond to a percentage of growth inhibition ((1-Abs_{600 nm} culture with Cabanillasin)/ Abs_{600 nm} culture without Cabanillasin) x 100.

The IC50 value is the concentration at which inhibition of growth is equal to 50%. MICs for *Candida* species were determined at 24 h and 48 h and corresponded to 100% of growth inhibition. Amphotericin B was provided as standard (final concentration ranging from 16 to 0.03 mg/L for all fungi). Experiments were done in duplicate.

### Cytotoxicity test

One hundred microliters of the cell suspension of PC-3 (Human prostatic carcinoma) or hTERT HME-1 (Human normal mammary epithelium) prepared in RPMI + 10% SVF + 1% glutamine were inoculated into 96-well plates. The inoculating cell number was 3500 cells per well for PC-3 and 7500 cells per well for hTERT HME-1. The microplates were incubated at 37°C for 24h with 5% of CO₂. After 24 h of culture, the medium was removed by aspiration and 100 µl of RPMI + 10% SVF + 1% glutamine with 8 concentrations of Cabanillasin (from 0.78 µg/mL to 100 µg/mL) were added to each well. Microplates were incubated at 37°C for 48 h with 5% of CO₂. After 48h of culture, the medium was removed by aspiration and 100 µL of RPMI + MTT (0.5 mg/mL) were added to each wells. Microplates were inoculated at 37°C during 150 min. Next, all the medium was removed by inverting and tapping the plate and 100 µL of DMSO were added in each plate. The spectrophotometric absorbance was then measured at 550 nm. The Lethal Dose 50 correspond to DL50 = ((50-(Y2-((Y1-Y2)/(X1-X2))*X2))/ ((Y1-Y2)/(X1-X2))). X1 represents the concentration for which viability was superior to 50%, X2 represents the concentration for which viability was inferior to 50%, Y1 represents the percentage of viability at the concentration X1 and Y2 the percentage of viability at the concentration X2. Experiments were done in triplicate.

### NMR and MS analysis

The purified compound was analyzed by Mass Spectroscopy and NMR to determine its chemical structure.

The NMR spectra were recorded in D₂O and DMSO-d6 on 5 mg of Cabanillasin. The experiments were recorded on a Bruker Avance spectrometer equipped with a cryoprobe at 500 MHz for ¹H and 126 MHz for ¹³C except for a set of ¹H and COSY NMR spectra in DMSO-d6 which were obtained on a Varian Gemini 300-BB spectrometer.

LC-MS was first performed in order to obtain the *m*/*z* value of the protonated Cabanillasin molecule. MS-MS fragmentation was then carried out. ESI-LC-MS data were obtained in the positive mode on a Waters alliance LC-MS system (Waters ZQ mass detector, Waters photodiode array detector 2696, Waters alliance HPLC systems 2790). The HPLC column used was a C18 column (Waters Symmetry C18 5µm 4.6X150 mm) maintained at 35 °C. Solvents were (A) water + 0.1% TFA (B) acetonitrile + 0.1% TFA and the flow rate was 1 mL/min. The mobile phase composition was 100% A at 0 min, ramped to 30% B at 30 min. Samples were dissolved in solvent A (100 µL). Sample injection volume was 10 µL. UV-Visible detection was by absorbance at 200-400 nm. Solvent flow to the MS was diverted to waste for the first 5 min to minimise salt build-up. MS-MS fragmentation data were obtained on a Waters Micromass Q-Tof micro mass spectrometer.

### B. Results

### Fermentation

*Xenorhabdus cabanillasii* CNCM I-4418 was cultivated for 48 h, at 28°C with shaking in a 2 L Erlenmeyer flask containing 500 mL of medium broth composed of bactopeptone 10 g/L, K₂HPO₄ 5 g/L, MgSO₄, 7H₂O 1 g/L, (NH₄)₂SO4 2g/L, CaCO₃ 2 g/L and NaCl 10 g/L. The culture was inoculated with 0.1% (v/v) of a 24 h preculture in the same medium. The antifungal production was controlled by analytical HPLC.

### Isolation

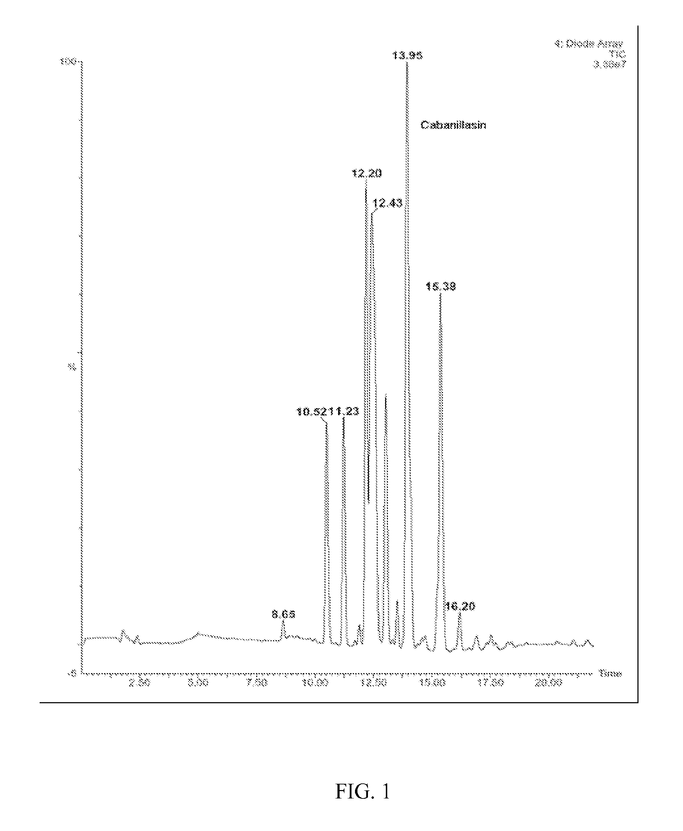

Bacterial cells were removed by low-speed centrifugation (6000 × g, 10 min at 4°C) and supernatant was sterilized on 0.22 µm pore size filter. Supernatant was added (1:1; v/v) to a 0.1 M NaCl-0.02 M Tris buffer (pH 7), and subjected to cation-exchange chromatography on a Sep Pack CarboxyMethyl cartridge (Acell^{™} Plus CM, Waters). Unbound material was removed by washes with a 0.1 M NaCl-0.02 M Tris buffer (pH 7) and the antibiotic activity eluted with 0.5 M NaCl-0.02 M Tris buffer (pH 7). This eluate was acidified with 0.1% (v/v) trifluoroacetic acid (TFA) and was then subjected to reversed-phase chromatography on a Sep Pack C18 cartridge (Sep-Pak plus C18, Waters). Unbound material was removed by washing with H₂O-TFA 0.1 % and the antibiotic pool was eluted with acetonitrile. The eluate was lyophilized then resuspended in water (1:5; v/v). Pure compounds were isolated from the crude extract by reverse phase HPLC using a C18 column (Waters ; Symmetry Symmetry C18; 5µm; 4.6X150 mm), a linear gradient of H₂O, 0.1% TFA-acetonitrile starting from 0% to 30% in 30 min, a flow rate of 1 mL/min and an UV detection from 200 to 400 nm, yielding pure Cabanillasin with the following HPLC-retention time: 13.95 min (Figure 1).

### Biological properties

Cabanillasin was tested for antifungal activity against a wide range of yeasts and filamentous fungi involved in nosocomial infection.

After 24 h of incubation, Cabanillasin demonstrates good activity against *Candida krusei* ATCC 6258 and *Candida lusitaniae* CBS 6936. Indeed, the IC50 values were respectively 6.25 µg/mL and 1.56 µg/mL against *Candida krusei* and *Candida lusitaniae* with MIC values of 25µg/mL. The inhibitory activity of this molecule against *Candida albicans* ATCC 90029 and *Candida Glabrata* ATCC 90030 was not complete with 20% to 40% of residual growth. The IC50 values against *Candida albicans* and *Candida glabrata* were respectively 0.78µg/mL and 6.25 µg/mL. After 48 h incubation, activity shows a significant decrease against yeasts. The instability of the inhibitory activity may be due to physico-chemical properties of Cabanillasin.

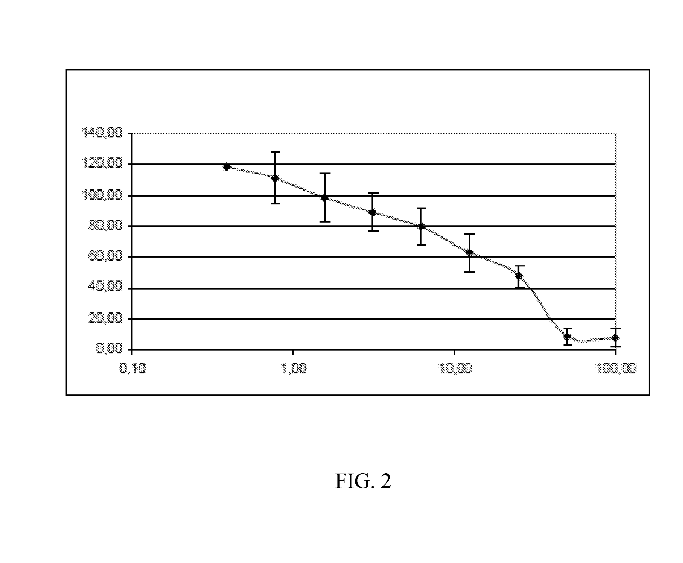

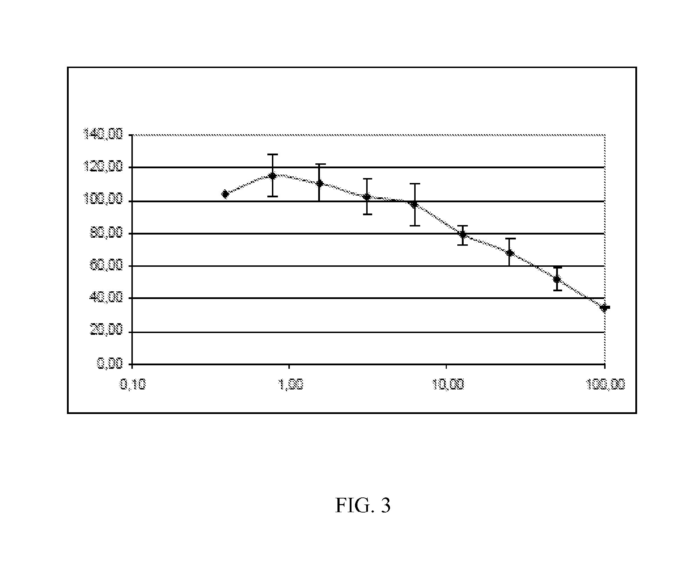

The inhibitory activity of Cabanillasin against *Cryptococcus neoformans* was moderate after 48 h (IC50 = 25µg/mL) and completely absent after 72 h (IC50 >50µg/mL). Regarding filamentous fungi, Cabanillasin have weak activity against *Aspergillus fumigatus* and *Rhizopus oryzae* at 48h and no activity at 72 h. On the other hand, the activity against *Fusrarium oxysporum* was interesting with IC50 value of 6.25 µg/mL. The LD 50 of Cabanillasin was 46.83±1.55 µg/mL against Human prostatic carcinoma cells (PC-3) and 24.46±2.51 µg/mL against Human normal mammary epithelium cells (hTERT HME-1) (Table 3). (Figures 2 and 3)

**Table 1: % of growth inhibition in presence of different Cabanillasin and Amphotericin B concentrations after 24 h incubation**

| | **Concentration of Cabanillisin in µg/mL** | | | | | | | | | | |
|---|---|---|---|---|---|---|---|---|---|---|---|
| | 50 | 25 | 12.5 | 6.25 | 3.12 | 1.56 | 0.78 | 0.39 | 0.19 | 0.09 | 0 |
| *C.albicans* | 81 | 71 | 70.7 | 47.7 | 45.4 | 46.3 | 50.5 | 34 | 37 | 34 | 0 |
| *C.glabrata* | 59 | 61.4 | 66.2 | 49.3 | 31 | 28 | 15.5 | 11.4 | 5.5 | 0 | 0 |
| *C.krusei* | 100 | 100 | 91 | 79 | 9 | 0 | 0 | 0 | 0 | 0 | 0 |
| *C.lusitaniae* | 100 | 100 | 97.7 | 94.7 | 86.7 | 64.8 | 20.2 | 11.5 | 14.5 | 11.7 | 0 |

| | **Concentration of Amphotericin B in µg/mL** | | | | | | | | | | |
|---|---|---|---|---|---|---|---|---|---|---|---|
| | 16 | 8 | 4 | 2 | 1 | 0.5 | 0.25 | 0.12 | 0.06 | 0.03 | 0 |
| *C.albicans* | 100 | 100 | 100 | 100 | 100 | 100 | 100 | 95.2 | 56.7 | 30.3 | 0 |
| *C.glabrata* | 100 | 100 | 100 | 100 | 100 | 100 | 100 | 100 | 71.3 | 16.8 | 0 |
| *C.krusei* | 100 | 100 | 100 | 100 | 100 | 100 | 100 | 100 | 48.4 | 0 | 0 |
| *C.lusitaniae* | 100 | 100 | 100 | 100 | 100 | 100 | 100 | 100 | 63 | 19 | 0 |

**Table 2: % of growth inhibition in presence of different Cabanillasin and Amphotericin B concentrations after 48 h incubation**

| | **Concentration of Cabanillisin in µg/mL** | | | | | | | | | | |
|---|---|---|---|---|---|---|---|---|---|---|---|
| | 50 | 25 | 12.5 | 6.25 | 3.12 | 1.56 | 0.78 | 0.39 | 0.19 | 0.09 | 0 |
| *C.albicans* | 18 | 0 | 0 | 0 | 0 | 0 | 0 | 0 | 0 | 0 | 0 |
| *C.glabrata* | 57 | 44 | 18.5 | 0 | 0 | 0 | 0 | 0 | 0 | 0 | 0 |
| *C.krusei* | 92.9 | 88.5 | 83 | 31.2 | 0 | 0 | 0 | 0 | 0 | 0 | 0 |
| *C.lusitaniae* | 100 | 95.9 | 86.1 | 74.6 | 57 | 0 | 0 | 0 | 0 | 0 | 0 |

| | **Concentration of Amphotericin B in µg/mL** | | | | | | | | | | |
|---|---|---|---|---|---|---|---|---|---|---|---|
| | 16 | 8 | 4 | 2 | 1 | 0.5 | 0.25 | 0.12 | 0.06 | 0.03 | 0 |
| *C.albicans* | 100 | 100 | 100 | 100 | 100 | 100 | 100 | 70 | 32 | 17 | 0 |
| *C.glabrata* | 100 | 100 | 100 | 100 | 100 | 100 | 100 | 68 | 23 | 25 | 0 |
| *C.krusei* | 100 | 100 | 100 | 100 | 100 | 100 | 100 | 76.7 | 48 | 25 | 0 |
| *C.lusitaniae* | 100 | 100 | 100 | 100 | 100 | 100 | 100 | 84.5 | 45.4 | 11.2 | 0 |

**Table 3: % of growth inhibition in presence of different Cabanillasin and Amphotericin B concentrations after 48 h incubation**

| | **Concentration of Cabanillisin in µg/mL** | | | | | | | | | | |
|---|---|---|---|---|---|---|---|---|---|---|---|
| | 50 | 25 | 12.5 | 6.25 | 3.12 | 1.56 | 0.78 | 0.39 | 0.19 | 0.09 | 0 |
| *Cr.neoformans* | 63.8 | 65.3 | 47.3 | 32 | 23.8 | 0 | 0 | 0 | 0 | 0 | 0 |
| *A.fumigatus* | 65.2 | 0 | 0 | 0 | 0 | 0 | 0 | 0 | 0 | 0 | 0 |
| *R.oryzae* | 40.7 | 0 | 0 | 0 | 0 | 0 | 0 | 0 | 0 | 0 | 0 |
| *F.oxysporum* | 80.2 | 66.6 | 71.6 | 68.6 | 32.1 | 0 | 0 | 0 | 0 | 0 | 0 |

| | **Concentration of Amphotericin in µg/mL** | | | | | | | | | | |
|---|---|---|---|---|---|---|---|---|---|---|---|
| | 16 | 8 | 4 | 2 | 1 | 0.5 | 0.25 | 0.12 | 0.06 | 0.03 | 0 |
| *Cr.neoformans* | 100 | 100 | 100 | 100 | 100 | 100 | 100 | 70 | 32 | 17 | 0 |
| *A.fumigatus* | 100 | 100 | 100 | 100 | 100 | 100 | 100 | 37.2 | 33.5 | 3 | 0 |
| *R.oryzae* | 100 | 100 | 100 | 100 | 92.2 | 47.4 | 43.9 | 22 | 35 | 26.7 | 0 |
| *F.oxysporum* | 100 | 100 | 100 | 100 | 100 | 100 | 65.2 | 39.8 | 26.3 | 14 | 0 |

**Table 4: % of growth inhibition in presence of different Cabanillasin and Amphotericin B concentrations after 72 h incubation**

| | **Concentration of Cabanillisin in µg/mL** | | | | | | | | | | |
|---|---|---|---|---|---|---|---|---|---|---|---|
| | 50 | 25 | 12.5 | 6.25 | 3.12 | 1.56 | 0.78 | 0.39 | 0.19 | 0.09 | 0 |
| *Cr.neoformans* | 0 | 0 | 0 | 0 | 0 | 0 | 0 | 0 | 0 | 0 | 0 |
| *A.fumigatus* | 0 | 0 | 0 | 0 | 0 | 0 | 0 | 0 | 0 | 0 | 0 |
| *R.oryzae* | 0 | 0 | 0 | 0 | 0 | 0 | 0 | 0 | 0 | 0 | 0 |
| *F.oxysporum* | 79.8 | 72.5 | 56.4 | 48.6 | 0 | 0 | 0 | 0 | 0 | 0 | 0 |

| | **Concentration of Amphotericin in µg/mL** | | | | | | | | | | |
|---|---|---|---|---|---|---|---|---|---|---|---|
| | 16 | 8 | 4 | 2 | 1 | 0.5 | 0.25 | 0.12 | 0.06 | 0.03 | 0 |
| *Cr.neoformans* | 100 | 100 | 100 | 100 | 100 | 100 | 81.5 | 44 | 5 | 0 | 0 |
| *A.fumigatus* | 100 | 100 | 100 | 100 | 100 | 100 | 85.2 | 41.5 | 0 | 0 | 0 |
| *R.oryzae* | 100 | 100 | 100 | 77.4 | 66 | 62.1 | 12 | 0 | 0 | 0 | 0 |
| *F.oxysporum* | 100 | 100 | 100 | 100 | 100 | 90.2 | 49.8 | 24.6 | 0 | 0 | 0 |

**Table 5: LD50 of Cabanillasin against PC-3 and hTERT HME-1**

| Cells | LD 50 in µg/ml | Standard deviation |
|---|---|---|
| PC-3 | 46.83 | 1.55 |
| hTERT HME-1 | 24.46 | 2.51 |

### Physico-chemical properties of Cabanillasin

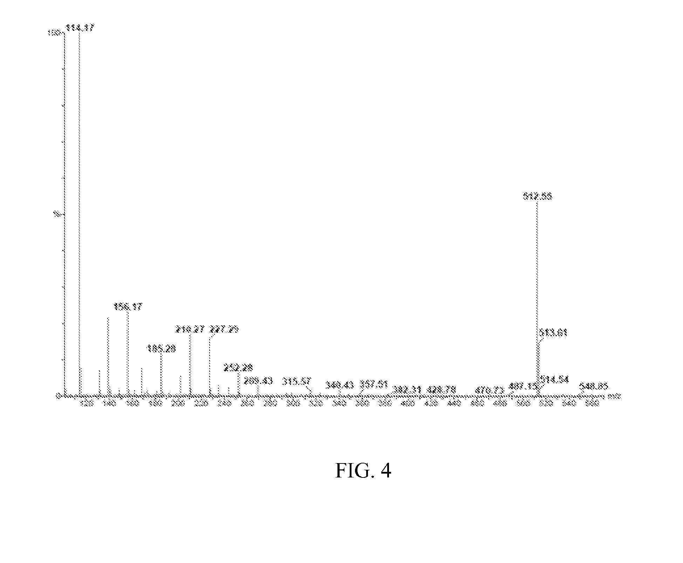

One compound referred to as Cabanillasin was isolated, purified to homogeneity as a white powder and characterized by mass spectrometry. ESI-MS experiments revealed the molecular weight of Cabanillasin (Figures 4)
Cabanillasin: White powder; UV λMeOHmax nm 214; ESI-MS (m/z) 512.5 [M+H]⁺

### Chemical structure elucidation

The ¹H NMR spectra showed 16 methylenes including 8 CH₂ bonded to nitrogen and an acetyl group. The ¹³C NMR spectra showed, in addition to signals corresponding to methylenes and methyl groups, a peak at 175 ppm confirming the presence of the acetyl group and 32 signals at 156 and 153 ppm characteristic of a quaternary carbon in a guanidine function. 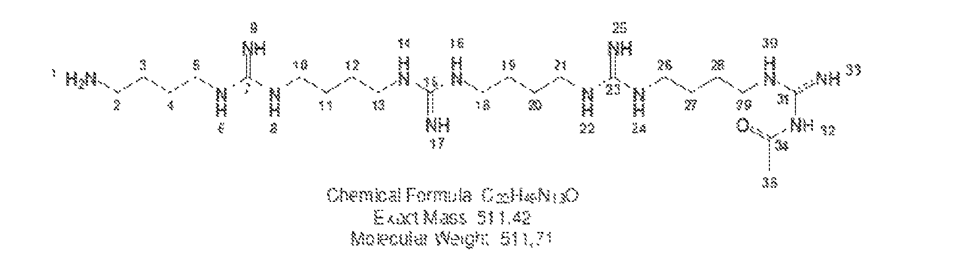 The assignment of ¹H and ¹³C in D₂O is
- ¹H NMR (500 MHz, D2O) δ 3.36 (2H, t, J=6.7 Hz, H-29), 3.28-3.16 (12H, m, H-5, 10, 13, 18, 21 and 26), 3.03 (2H, t, *J*= 7.3Hz, H-2), 2.24 (3H, s, H-35), 1.80-1.53 (16H, m, H-3, 4, 11, 12, 19, 20, 27 and 28).
- 13C NMR (126 MHz, D2O) δ 174.79 (s, C-34), 155.72 (s, C-7, 15 and 23), 152.93(s, C-31), 41.01-40.44 (m, C-5, 10, 13, 18, 21, 26 and 29), 39.06 (s, C-2), 25.42-23.86 (m, C-3, 4, 11, 12, 19, 20, 27 and 28), 23.7 (s, C-35).

The assignment of ¹H and ¹³C in DMSO-d6 is
- ¹H NMR (500 MHz, DMSO) δ 7.91-7.60 (8H, m, NH), 7.53-7.33 (6H, m, NH), 3.27 (2H, t, *J*= 6.7 Hz, H-29), 3.14-3.08 (12H, m, H-5, 10, 13, 18, 21 and 26), 2.80 (2H, t, *J*=6.8 Hz, H-2), 2.12 (3H, s, H-35), 1.61-1.41 (16H, m, H-3, 4, 11, 12, 19, 20, 27 and 28).
- ¹³C NMR (126 MHz, DMSO) δ 173.16 (s, C-34), 155.60 (s, C-7, 15 and 23), 153.47(s, C-31), 40.86-40.20 (m, C-5, 10, 13, 18, 21, 26 and 29), 38.41 (s, C-2), 26.11-24.75 (m, C-3, 4, 11, 12, 19, 20, 27 and 28), 24.24 (s, C-35).

In this study, we have described the discovery of a new antifungal family - the Cabanillasins - produced by fermentation of a *Xenorhabdus cabanillasii* strain, and purified by cation exchange chromatography and reversed phase chromatography. The chemical structure of Cabanillasin was obtained from the analysis of homo and heteronuclear 2D NMR and MS-MS experiments. Cabanillasin showed activity against yeasts and filamentous fungi involved in hospital-acquired infection.

### REFERENCES

Clinical Microbiology Reviews, January 2007, p. 133-163, Vol. 20, No. 1
Clinical infectious diseases 2002; 35, 1073-80
J. Nat. Prod. 54, 785-95 (1991)
J. Nat. Prod. 58, 1081-6 (1995)
Can. J. Microbiol. 43, 770-3 (1997)
J.Antibiot. 62(6) 295-302 (2009)

### PATENT REFERENCES

WO 84/01775
US 5,569,668

## Claims

1. Compound of formula (I): 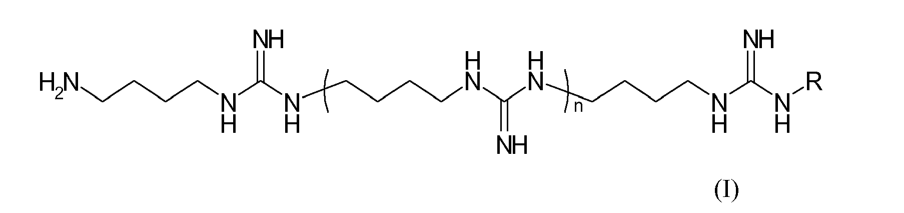 wherein
n is 1, 2, 3 or 4,
R is selected in the group consisting of H, C(=O)-CH₃, C(=O)-CH₂-CH₃ and C(=O)-CH(-CH₃)-CH₃.

2. Compound according to claim 1 wherein R is C(=O)-CH₃.

3. Compound according to anyone of claims 1-2 according to formula (II):

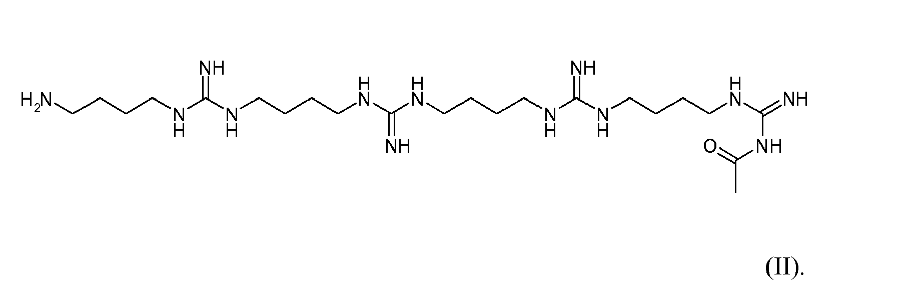

4. Compound according to anyone of claims 1-3 for use as a medicament.

5. Compound according to anyone of claims 1-4 for use as an antifungal agent.

6. Compound according to anyone of claims 1-5 for use in treatment of fungal disease caused by yeast and/or filamentous fungi.

7. Compound according to anyone of claims 1-6 for use in treatment of yeast and/or fungal disease caused by a pathogen selected from *Candida*, *Pneumocystis*, *Cryptococcus*, *Trichosporon*, *Aspergillus*, *Exophiala, Fonsecaea*, *Phialophora*, *Geotrichum, Pseudallescheria, Fusarium, Rhizopus*, *Epidermophyton, Microsporum*, *Trichophyton*, *Coccidioides* and *Histoplasma*.

8. Compound according to anyone of claims 1-7 for use in treatment of yeast and/or fungal disease caused by a pathogen selected from *C.albicans*, *C.glabrata*, *C.krusei*, *C.lusitaniae*, *Cr.neoformans*, *A.fumigatus*, *R.oryzae* and *F.oxysporum*.

9. Pharmaceutical composition comprising:
a. An effective amount of a compound of formula (I) 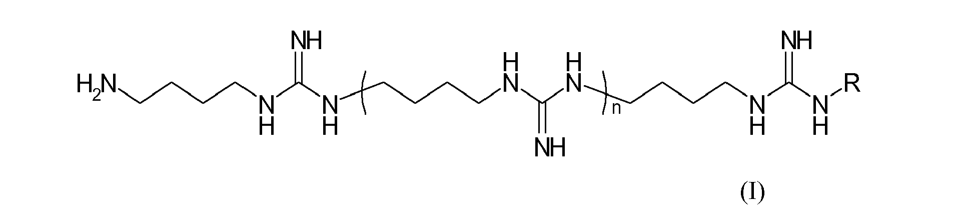 wherein
n is 1, 2, 3 or 4,
R is selected in the group consisting of H, C(=O)-CH₃, C(=O)-CH₂-CH₃ and C(=O)-CH(-CH₃)-CH₃,
b. A pharmaceutically acceptable carrier.

10. Method for producing a compound according to formula (II) comprising the following steps
a. Growing *Xenorhabdus cabanillasii* strain CNCM I-4418 in a liquid culture medium,
b. Purifying a compound according to formula (II)

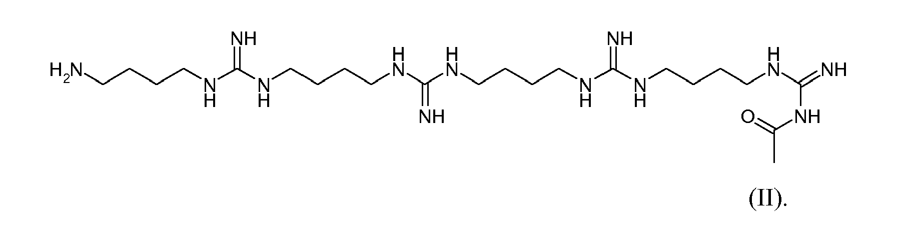

11. Method according to claim 10 wherein the compound of formula (II) is purified from the culture supernatant after removal of the *Xenorhabdus cabanillasii* cells.

12. *Xenorhabdus cabanillasii* strain deposited at CNCM having the accession number CNCM I-4418.

13. Culture supernatant from the *Xenorhabdus cabanillasii* strain of claim 12 having antifungal activity.

14. Extract from the *Xenorhabdus cabanillasii* strain of claim 12 having antifungal activity.
